# FASCICULE DE BREVET EUROPEEN

(11) **EP 3 718 522 B1**
(45) Date de publication et mention de la délivrance du brevet: **22.07.2026**
(21) Numéro de dépôt: 20165909.1
(22) Date de dépôt: 26.03.2020
(51) Int. Cl.: A61F 11/06, A61F 13/12, A61F 13/02

(54) **NÉCESSAIRE POUR LA PROTECTION DE L'OREILLE**
SET ZUM SCHUTZ DER OHREN
KIT FOR EAR PROTECTION

(30) Priorité: 02.04.2019 FR 1903528
(43) Date de publication de la demande: 07.10.2020
(73) Titulaire: Douchossec, 13400 Aubagne (FR)
(72) Inventeur: GILLI, Eric, 13260 CASSIS (FR)
(74) Mandataire: Cyrson, Matthew Dominic

(56) Documents cités:
- EP-A1- 2 612 630
- WO-A1-2004/000426
- WO-A1-2006/100382
- FR-A1- 3 018 453
- US-A- 1 826 309
- US-A1- 2012 124 719
- US-A1- 2016 192 764

## Description

### Domaine de l'invention :

La présente invention concerne un nécessaire pour la protection de l'oreille d'un patient.

### Art antérieur :

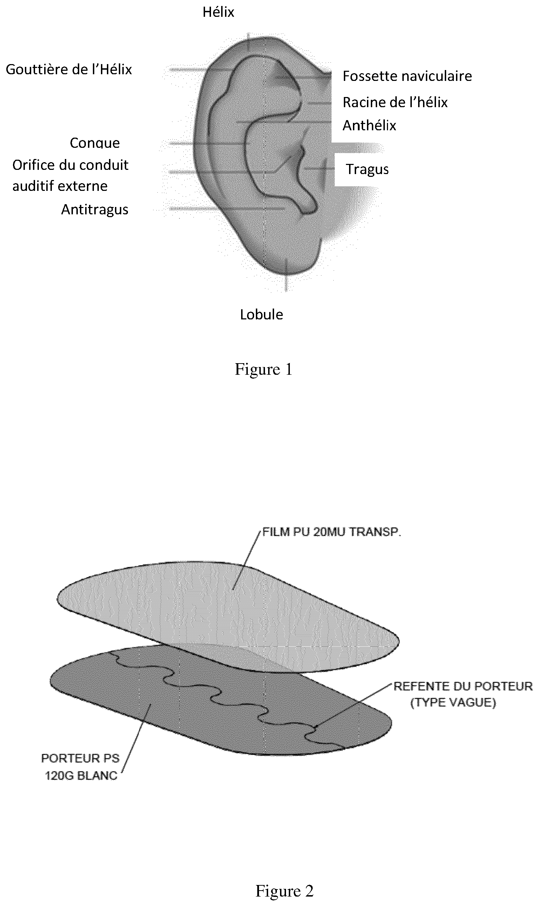

Le pavillon de l'oreille (figure 1) qui constitue la seule partie externe visible est pour l'essentiel composé de cartilage flexible et élastique, recouvert de peau sur ses deux surfaces. La seule partie non composée de cartilage est le lobule, petite extrémité de l'oreille, plus communément appelée lobe. Ce pavillon, qui a une forme de coquille, est destiné à la réception et à la concentration des ondes sonores. Pour se faire, ce pavillon est composé de nombreux muscles et recoins pour mieux capter ces sons, afin d'en envoyer le maximum vers l'étape usuelle suivante d'une onde, le conduit auditif externe. Si les muscles de l'oreille n'ont pas un réel effet chez l'Homme, on les remarque cependant chez les animaux.

Du fait de cette morphologie complexe, la protection de l'oreille de façon fiable est rendue difficile.

Toutefois, cette protection est souvent rendue nécessaire lors d'otites « estivales » ou plus chroniques. En effet, on pose souvent chez les enfants présentant des otites à répétition des dispositifs dit « yoyo » ou diabolos qui sont des aérateurs transtympaniques. Il s'agit en fait de drains permettant d'aérer en permanence la caisse du tympan et d'éviter la récidive des otites.

Maintenant, il est fortement recommandé de ne pas se baigner après la pose de tels dispositifs. En effet, l'eau est alors susceptible de provoquer une inflammation ou une infection. Les problèmes d'otite affectant les enfants en bas âge, il est toutefois difficile de faire respecter les recommandations et une vigilance de chaque instant est donc nécessaire.

### Sommaire de l'invention :

L'inventeur a maintenant développé un nécessaire permettant de protéger simplement l'oreille.

En conséquence, un premier objet de l'invention est un nécessaire pour la protection de l'oreille d'un patient selon la revendication 1, comprenant :
- Au moins une feuille de couverture souple non encollée en matière synthétique souple qui est à base de polyuréthane, destinée à être appliquée sur l'oreille ; et
- Au moins un conditionnement contenant une substance adhésive qui comprend un copolymère à base de silicone présentant un orifice de sortie permettant la pause d'un cordon de cette substance adhésive qui relie le contour du pavillon de l'oreille du patient et la feuille de couverture ; la feuille de couverture présentant une forme ovoïde avec une longueur comprise entre 7 et 9 cm, une largeur comprise entre 5 et 7 cm ; et une épaisseur comprise entre 10 et 100 µm.

### Description des figures :

La figure 1 représente le pavillon de l'oreille
Les figures 2, 3 et 4 représentent des vues schématiques d'un exemple de feuille de protection associée à une feuille de couverture

### Description détaillée de l'invention :

La demande internationale WO2006/100382 divulgue un dispositif permettant de protéger une surface de peau, comprenant une feuille de couverture souple non encollée en matière synthétique souple qui de péférence est à base de polyuréthane et presentant, de préférence, une épaisseur comprise entre 10 µm et 80 µm, et un tube contenant un adhésif avantageusement constitué par un copolymère, de préférence à base de silicone. Rien ne permettait d'envisager l'adaptation du nécessaire décrit à la protection de l'oreille d'un patient. Les documents US2016/192764, US2012/124719 et US61826309 divulguent des protections des oreilles. FR3018453 décrit un nécessaire pour le maintien et la protection de cathéters placés sur une zone du corps d'un patient. EP2612630 divulgue un patch cutané adhésif.

La matière synthétique de la feuille de couverture souple confère des propriétés d'étanchéité à l'eau et est en outre étanche non allergénique.

A titre d'exemple de matériau utilisable, on pourra citer les matières à base de polyuréthane, de polypropylène ou de polyéthylène. Selon la présente invention on utilisera du polyuréthanne ou un polymère à base de polyuréthane.

Maintenant, cette feuille de couverture pourra être transparente ou opaque, de préférence transparente de sorte de pouvoir observer facilement l'état de l'oreille sans avoir à l'ôter.

L'épaisseur de la feuille de couverture doit être suffisante, en fonction de sa composition, pour permettre une bonne étanchéité et une bonne résistance aux chocs. Typiquement, cette épaisseur devra être comprise entre 10 µm et 50 µm et, de préférence de 20 µm pour une feuille de couverture en polyuréthane.

Pour ce qui est de la forme, elle est ovoïde, c'est-à-dire qu'elle correspond à celle d'un œuf. Selon la présente invention la forme de la feuille de couverture est de type ovoïde écrasé Idéalement, la feuille de couverture présente :
- une longueur comprise entre 7 et 9 cm, de préférence entre 7,5 et 8,5 cm et, de manière particulièrement préférée entre 7,9 et 8,1 cm ; et
- une largeur comprise entre 5 et 7 cm, de préférence entre 5,5 et 6,5 cm, et de manière particulièrement préférée, entre 6 et 6,2 cm.

L'inventeur a ainsi mis en évidence que de telles dimensions permettent très simplement de s'adapter à tout type d'oreille.

La feuille de couverture est non encollée ce qui lui confère une meilleure perméabilité à l'air et facilite le passage des ondes sonores même si elle les atténue.

En outre, le nécessaire présente avantageusement une feuille de protection détachable appliquée contre la face externe de la feuille de couverture, cette feuille de protection présentant une souplesse inférieure à la feuille de couverture.

Pour faciliter son retrait, cette feuille de protection présente avantageusement une découpe, laquelle découpe facilite son retrait. Dans l'idéal, cette découpe est positionnée dans la longueur et au milieu de la feuille de protection. A noter qu'elle peut être droite ou en forme de vague.

En ce qui concerne le conditionnement contenant une substance adhésive, celui-ci peut prendre des formes multiples, il peut ainsi prendre la forme d'un tube compressible ou encore d'une cartouche rigide, pressurisée ou non.

Dans le cas où le conditionnement contenant une substance adhésive prend la forme d'un tube compressible, celui-ci comprend alors une tête d'extrusion ou un goulot agencé, de manière connue en soi, pour permettre la distribution de la substance adhésive de sorte qu'elle forme un cordon de largeur homogène et, préférentiellement, souple.

De préférence, la tête d'extrusion ou le goulot est agencé pour permettre la distribution de la substance adhésive de sorte qu'elle forme un cordon de largeur égale ou inférieure à 5 mm, de préférence de largeur égale ou inférieure à 4 mm voir à 3 mm et, de manière particulièrement préférée, de largeur inférieure ou égale à 2 mm.

La substance adhésive peut prendre, par exemple, la forme d'un liquide, d'une mousse ou d'un gel, de préférence d'un gel.

Cette substance adhésive, une fois appliquée, permet l'adhérence de ladite au moins une feuille de couverture à la surface de peau et de garantir l'étanchéité à l'eau du volume ainsi délimité (surface de peau entourée de substance adhésive et recouverte d'au moins une feuille de couverture).

Cette substance adhésive est également non allergénique et biocompatible.

Idéalement, cette substance adhésive peut présenter des propriétés d'adhérence à la peau moins fortes que ses propriétés d'adhérence à la feuille de couverture ; ceci de sorte à permettre le retrait de la feuille de couverture sans effet douloureux ni résidus de substance adhésive sur la peau.

Idéalement toujours, les propriétés d'adhérences de la substance adhésive sont telles qu'elles permettent le maintien de la feuille de couverture sur la peau du patient pendant un voir plusieurs jours.

Cette substance adhésive comprend avantageusement un homopolymère ou un copolymère. Idéalement, cette substance adhésive comprend un copolymère, de préférence à base de silicone, comme par exemple une colle à base de polydiméthylsiloxane, lequel copolymère peut comprendre en outre des groupes fonctionnels.

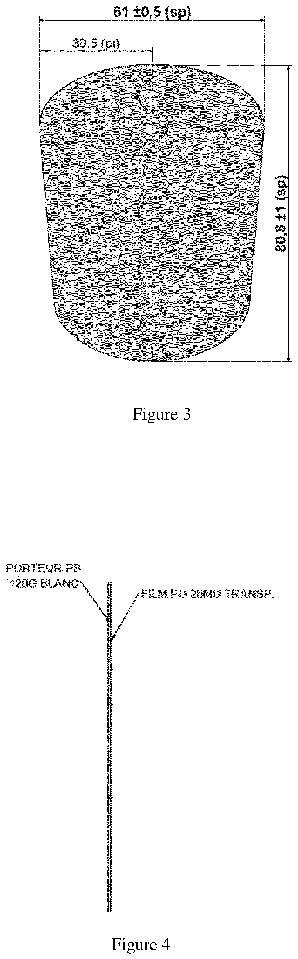

Les figures 2, 3 et 4 montrent un mode de réalisation préféré dans lequel le nécessaire intègre une feuille de couverture en polyuréthane

## Revendications

1. Un nécessaire pour la protection de l'oreille d'un patient comprenant :
- une feuille de couverture souple non encollée en matière synthétique souple qui est à base de polyuréthane, qui est destinée à être appliquée sur l'oreille et qui présente
- une longueur comprise entre 7 et 9 cm,
une largeur comprise entre 5 et 7 cm ; et
- Au moins un conditionnement contenant une substance adhésive qui comprend un copolymère à base de silicone présentant un orifice de sortie permettant la pause d'un cordon de cette substance adhésive qui relie le contour du pavillon de l'oreille du patient et la feuille de couverture ;
où la feuille de couverture présente une forme de type ovoïde écrasé et une épaisseur comprise entre 10 et 100 µm.

2. Le nécessaire selon la revendication 1, **caractérisé en ce que** l'épaisseur de la feuille de couverture est comprise entre 10 µm et 50 µm.

3. Le nécessaire selon l'une quelconque des revendications 1 ou 2, **caractérisé en ce que** l'épaisseur de la feuille de couverture est de 20 µm.

4. Le nécessaire selon l'une quelconque des revendications 1 à 3, **caractérisé en ce que** la substance adhésive est une colle à base de polydiméthylsiloxane.

5. Le nécessaire selon l'une quelconque des revendications 1 à 4, **caractérisé en ce que** la feuille de couverture présente
- une longueur comprise entre 7,5 et 8,5 cm et, de manière particulièrement préférée entre 7,9 et 8,1 cm ; et
- une largeur comprise entre 5,5 et 6,5 cm, et de manière particulièrement préférée, entre 6 et 6,2 cm.

6. Le nécessaire selon l'une quelconque des revendications 1 à 5, **caractérisé en ce que** le nécessaire présente en outre une feuille de protection détachable appliquée contre la face externe de la feuille de couverture, laquelle feuille de protection présente une souplesse inférieure à la feuille de couverture.

7. Le nécessaire selon l'une quelconque des revendications 1 à 6, **caractérisé en ce que** la feuille de protection présente une découpe.

8. Le nécessaire selon l'une quelconque des revendications 1 à 7, **caractérisé en ce que** le conditionnement prend la forme d'un tube compressible.

9. Le nécessaire selon la revendication 8, **caractérisé en ce que** le tube compressible comprend une tête d'extrusion ou un goulot agencé pour permettre la distribution de la substance adhésive de sorte qu'elle forme un cordon de largeur égale ou inférieure à 5 mm, de préférence de largeur égale ou inférieure à 4 mm voir à 3 mm et, de manière particulièrement préférée, de largeur inférieure ou égale à 2 mm.

## Patentansprüche

1. Ein Kit zum Schutz des Ohrs eines Patienten, umfassend:
- eine nicht-adhäsive, flexible Abdeckfolie
aus einem flexiblen Kunststoffmaterial auf Polyurethanbasis,
die dazu bestimmt ist, über dem Ohr aufgebracht zu werden,
- und eine Länge zwischen 7 und 9 cm
sowie eine Breite zwischen 5 und 7 cm
aufweist; und
- mindestens eine Verpackungseinheit, die eine Klebemasse enthält, welche ein Copolymer auf Silikonbasis umfasst und eine Austrittsöffnung aufweist die das Aufbringen einer Raupe dieser Klebesubstanz ermöglicht, um den Rand der Ohrmuschel des Patienten mit der Abdeckfolie zu verbinden;
wobei die Abdeckfolie
eine abgeflachte, ovale Form und eine Dicke zwischen 10 und 100 µm aufweist.

2. Kit nach Anspruch 1, **dadurch gekennzeichnet, dass** die Dicke der Abdeckfolie zwischen 10 µm und 50 µm beträgt.

3. Kit nach Anspruch 1 oder Anspruch 2, **dadurch gekennzeichnet, dass** die Dicke der Abdeckfolie 20 µm beträgt.

4. Kit nach Anspruch 1 oder Anspruch 3,
**dadurch gekennzeichnet, dass** die Klebemasse
ein Klebstoff auf Polydimethylsiloxanbasis ist.

5. Kit nach einem der Ansprüche 1 bis 4,
**dadurch gekennzeichnet, dass** die Abdeckfolie
- eine Länge zwischen 7,5 und 8,5 cm und, besonders bevorzugt, zwischen 7,9 und 8,1 cm aufweist; sowie
- eine Breite zwischen 5,5 und 6,5 cm und, besonders bevorzugt, zwischen 6,0 und 6,2 cm aufweist.

6. Kit nach einem der Ansprüche 1 bis 5,
**dadurch gekennzeichnet, dass** der Kit ferner eine abziehbare Schutzfolie umfasst, die auf die Außenfläche
der Abdeckfolie aufgebracht ist, wobei diese Schutzfolie eine geringere Flexibilität als die Abdeckfolie aufweist.

7. Kit nach einem der Ansprüche 1 bis 6,
**dadurch gekennzeichnet, dass** die Abdeckfolie eine Aussparung aufweist.

8. Kit nach einem der Ansprüche 1 bis 7,
**dadurch gekennzeichnet, dass** die Verpackungseinheit die Form einer ausdrückbaren Tube annimmt.

9. Kit nach Anspruch 8, **dadurch gekennzeichnet, dass** die ausdrückbare Tube einen Extrusionskopf oder einen Hals aufweist, der dazu ausgelegt ist, die Klebemasse
so auszubringen, dass sie eine Raupe mit einer Breite von gleich oder weniger als 5 mm bildet,
vorzugsweise mit einer Breite von gleich oder weniger als 4 mm oder 3 mm und, besonders bevorzugt, mit einer Breite von gleich oder weniger als 2 mm.

## Claims

1. A kit for protecting a patient's ear, comprising:
- a non-adhesive flexible cover sheet made
of a flexible polyurethane-based synthetic material, intended to be applied over the ear
- and having a length between 7 and 9
cm, a width between 5 and 7 cm; and
- At least one package containing an adhesive substance that comprises a silicone-based copolymer,
featuring an outlet orifice allowing for the application of a bead of this adhesive substance to connect a periphery of the patient's auricle and the cover sheet;
wherein said cover sheet
has a flattened ovoid shape and a thickness between 10 and 100 µm.

2. The kit as claimed in claim 1, **characterized in that** the thickness of the cover sheet is between 10 µm and 50 µm.

3. The kit as claimed in claim 1 or claim 2, **characterized in that** the thickness of the cover sheet is 20 µm.

4. The kit as claimed in claim 1 or claim 3,
**characterized in that** the adhesive substance is a polydimethylsiloxane-based glue.

5. The kit as claimed in any one of the claims 1 to 4,
**characterized in that** the cover sheet has
- a length between 7.5 and 8.5 cm and, particularly preferably, between 7.9 and 8.1 cm; and
- a width between 5.5 and 6.5 cm and, particularly preferably, between 6 and 6.2 cm.

6. The kit as claimed in any one of the claims 1 to 5,
**characterized in that** the kit further comprises a detachable protective sheet applied against the external face of the cover sheet, wherein such protective sheet has a flexibility lower than the cover sheet.

7. The kit as claimed in any one of the claims 1 to 6,
**characterized in that** the cover sheet has a cutout.

8. The kit as claimed in any one of the claims 1 to 7,
**characterized in that** the package takes the form of squeezable tube

9. The kit as claimed in claim 8, **characterized in that** the
squeezable tube comprises an extrusion head or a neck arranged to allow the distribution of the adhesive substance
such that it forms a bead with a width equal to or less than 5 mm
preferably with a width equal to or less than 4 mm or 3 mm and, particularly preferably, with a width less than or equal to 2 mm.
